# FASCICULE DE BREVET EUROPEEN

(11) **EP 0 900 074 B1**
(45) Date de publication et mention de la délivrance du brevet: **15.05.2002**
(21) Numéro de dépôt: 97915541.3
(22) Date de dépôt: 21.03.1997
(51) Int. Cl.: A61K 7/13

(54) **COMPOSITIONS DE TEINTURE DES FIBRES KERATINIQUES CONTENANT DES PYRAZOLIN-3,5-DIONE; LEUR UTILISATION POUR LA TEINTURE COMME COUPLEURS, PROCEDE DE TEINTURE**
ZUSAMMENSETZUNG ZUR FÄRBUNG VON KERATINISCHEN FASERN ENTHALTEND PYRAZOLIN-3,5-DION ALS KUPPLER, UND FÄRBEVERFAHREN
KERATIN FIBRE DYE COMPOSITIONS CONTAINING PYRAZOLIN-3,5-DIONE COMPOUNDS, USE THEREOF AS DYE COUPLERS, AND DYEING METHOD

(30) Priorité: 22.03.1996 FR 9603630
(43) Date de publication de la demande: 10.03.1999
(73) Titulaire: L'OREAL, 75008 Paris (FR)
(72) Inventeur: VIDAL, Laurent, F-75013 Paris (FR); MALLE, Gérard, F-77100 Meaux (FR)
(74) Mandataire: Miszputen, Laurent
(86) Numéro de dépôt international: FR9700509
(87) Numéro de publication internationale: WO9735553

(56) Documents cités:
- DE-A- 2 160 317
- FR-A- 1 564 999
- US-A- 3 820 948

## Description

L'invention a pour objet une composition pour la teinture par oxydation des fibres kératiniques en particulier des cheveux humains contenant au moins un composé pyrazolin-3,5-dione comme coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphényiènediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet, différemment sensibilisée (i.e. abîmée) entre sa pointe et sa racine.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des composés pyrazolin-3,5-dione comme coupleurs en présence d'une base d'oxydation.

Cette découverte est à la base de la présente invention.

L'invention a pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un composé pyrazolin-3,5-dione de formule (I) ou l'un de ses sels d'addition avec un acide : 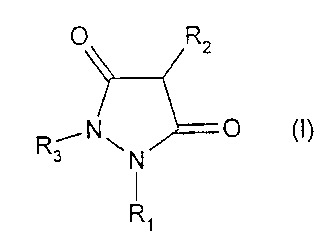
dans laquelle :
. R₂ représente : un atome d'hydrogène ; un atome d'halogène tel que brome, chlore ou fluor ; un groupe acétylamido ; un radical alcoxy (tel que par exemple : méthoxy, éthoxy, propyloxy, benzyloxy, méthoxyéthoxy, phénoxyéthoxy, 2-cyanoéthoxy, phénéthyloxy, p-chiorobenzyloxy, méthoxyéthylcarbamoylméthoxy); un radical aryloxy (tel que par exemple : phénoxy, 4-méthoxyphénoxy, 4-nitrophénoxy, 4-cyanophénoxy, 4-méthanesulfonamidophénoxy, 4-méthanesulfonylphénoxy, 3-méthylphénoxy, 1-naphtyloxy) ; un radical acyloxy (tel que par exemple : acétoxy, propanoyloxy, benzoyloxy, 2,4-dichlorobenzoyloxy, éthoxyalkyloxy, pyruviloyloxy, cinnamoyloxy, myristoyloxy) ; un radical arylthio (tel que par exemple : phénylthio, 4-carboxy-phénylthio, 2-éthoxy 5-tert-butylphénylthio, 2-carboxyphénylthio, 4-méthane-sulfonyl-phénylthio) ; un radical alkylthio (tel que par exemple : méthylthio, éthylthio, propylthio, butylthio, 2-cyanoéthylthio, benzylthio, phénéthylthio, 2-(diéthylamino) éthylthio, éthoxyéthylthio, phénoxyéthylthio) ; un radical hétéroarylthio (tel que par exemple : 5-phényl 2,3,4,5-tétrazolylthio, 2-benzothiazolylthio) ; un radical hétéroaryloxy (tel que par exemple : 5-phényl 2,3,4,5-tétrazolyloxy, 2-benzo-thiazolyloxy); un radical thiocyano ; un radical N,N-diéthyl thiocarbonylthio ; un radical dodécyl-oxythio carbonylthio ; un radical benzènesulfonamido ; un radical N-éthyltoluéne sulfonamido ; un radical pentafluorobutanamido ; un radical 2,3,4,5,6-pentafluorobenzamido ; un radical p-cyanophényluréido, un radical N,N-diéthyl-sulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; un radical NR^{III}R^{IV} avec R^{III} et R^{IV} représentant, identiques ou différents, un alkyle en C₁-C₄ , un hydroxyalkyle; un carboxyle ; ou un radical alcoxycarboxylique.
. R₁ et R₃ représentent un atome d'hydrogène ; un radical alkyle en C₁-C₅ linéaire ou ramifié ; un radical mono- ou poly-hydroxyalkyle en C₂-C₄ ; un radical aminoalkyle en C₂-C₄ ; un radical phényle ; un radical phényle substitué par un atome d'halogène ou un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, méthylènedioxy ou amino ; ou un radical 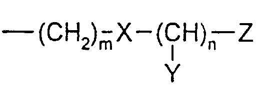 dans lequel m et n sont des nombres entiers, identiques ou différents, compris entre 1 et 3 inclusivement, X représente un atome d'oxygène ou bien le groupement NH, Y représente un atome d'hydrogène ou bien un radical méthyle, et Z représente un radical méthyle, un groupement OR ou NRR' dans lesquels R et R', qui peuvent être identiques ou différents, désignent un atome d'hydrogène, un radical méthyle ou un radical éthyle ; un radical amino; un alkylamino en C₁-C₄ ; un radical carboxyle ; un radical alcoxycarbonyle ; un radical aryloxycarbonyle ; un hétérocycle à 5 ou 6 chaînons possédant au moins un atome d'azote, de soufre ou d'oxygène (tel que pyridyle, quinolyle, pyrrolyle, morpholyle, furanyle, pyrazolyle, triazolyle, tétrazolyle, thiazolyle, oxazolyle, imidazolyle, thiadiazolyle); un radical acyle ; un groupe sulfonyle; un groupe phosphonyle;
étant entendu que lorsque R₂ et R₃ représentent simultanément un atome d'hydrogène, alors R₁ est différent d'un atome d'hydrogène, d'un radical alkyle en C₁-C₅ ou d'un radical phényle ;
- et au moins une base d'oxydation.

Les sels d'addition avec un acide des composés de l'invention peuvent être choisis notamment parmi des chlorhydrates, les bromhydrates, les tartrates, les tosylates, les benzènesulfonates, les sulfates, les lactates et les acétates.

Parmi les radicaux R₂ de la formule (I) définie ci-dessus, on préfère les radicaux choisis dans le groupe constitué par :
un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido; un radical NR^{III}R^{IV} avec R^{III} et R^{IV} représentant, identiques ou différents, un alkyle en C₁-C₄ , un hydroxyalkyle en C₁-C₄ ; un carboxyle ; un radical alcoxycarboxylique en C₁-C₄.

Parmi les radicaux R₂ de la formule (I) définie ci-dessus, on préfère plus particulièrement les radicaux choisis dans le groupe constitué par :
hydrogène ; chlore ou brome ; méthoxy ou éthoxy ; phénoxy ; 4-méthylphénoxy ; acyloxy ; benzyloxy ; méthylthio ou éthylthio ; phénylthio ; 4-méthylphénylthio ; 2-tertio-butylphénylthio ; acétamido ; phénylacétamido ; diméthylamino ; diéthylamino ; éthyl-méthylamino ; (β-hydroxyéthyl) méthylamino.

Et encore plus particulièrement , on préfère les radicaux R₂ choisis dans le groupe constitué par : hydrogène ; chlore ; éthoxy ; phénoxy ; benzyloxy ; acyloxy ; acétamido ; diméthylamino.

Parmi les radicaux R₁ et R₃ de la formule (I) définie ci-dessus, on préfère les radicaux choisis dans le groupe constitué par : hydrogène ; alkyle en C₁-C₄ (tel que méthyle, éthyle, isopropyle, t-butyle, n-propyle) ; mono ou polyhydroxyalkyle en C₂-C₄ (tel que 2-hydroxyéthyle, 3,4-dihydroxybutyle) ; aminoalkyle en C₂-C₄ (tel que 2-aminoéthyle) ; dialkylaminoalkyle (tel que 2-(N,N-diméthylamino)éthyle) ; phényle ; phényle substitué par un atome de chlore, un radical méthoxy, nitro, trifluorométhyle, amino, méthylamino ou méthyle ; benzyle ; benzyle substitué par un chlore, un méthoxy ou un méthyle; alcoxycarbonyle (tel que méthoxycarbonyle, éthoxycarbonyle) ; aryloxycarbonyle (tel que phényloxycarbonyle) ; pyridyle ; furyle ; thiènyle ; pyrrolyle ; thiazolyle ; acyle (tel que acétyle, 2-éthylcarbonyle).

Parmi les radicaux R₁ et R₃, on préfère plus particulièrement les radicaux choisis dans le groupe constitué par :
hydrogène ; méthyle ; éthyle ; isopropyle ; 2-hydroxyéthyle ; 2-aminoéthyle ; phényle ; 2-, 3-, ou 4-chlorophényle ; 3 ou 4-méthoxyphényle ; benzyle ; 3 ou 4-toluyle ; méthoxycarbonyle ; éthoxycarbonyle ; pyridyle ; pyrazolyle ; pyrrolyle.

Et encore plus particulièrement, on préfère les radicaux R₁ et R₃ choisis dans le groupe constitué par :
hydrogène ; méthyle ; éthyle ; isopropyle ; phényle ; 4-chlorophényle ; 4-toluyle ; benzyle ; pyridyle ; pyrazolyle.

Les composés de formule (I) particulièrement préférés sont ceux pour lesquels :
R₁ désigne hydrogène, méthyle, éthyle ou phényle;
R₂ désigne chlore ou éthoxy;
R₃ désigne méthyle, éthyle ou phényle.

A titre de composés de formule (I) ci-dessus, on peut tout particulièrement citer :
- la 1,2-diphényl pyrazolin-3,5-dione,
- la 1,2-diéthyl pyrazolin-3,5-dione,
- la 1,2-diméthyl pyrazolin-3,5-dione,
- la 4-chloro-1,2-diéthyl pyrazolin-3,5-dione,
et leurs sels d'addition avec un acide.

Les composés pyrazolin-3,5-diones de l'invention, leurs intermédiaires de synthèse et leurs procédés de préparation sont décrits dans les demandes de brevet et brevets JP 07-036159, JP 07-084348 et US 4 128 425, et dans les publications suivantes :
- L. WYZGOWSKA, Acta. Pol. Pharm. 1982, 39 (1-3), 83.
- E. HANNIG, Pharmazie, 1980, 35 (4), 231
- M. H. ELNAGDI, Bull. Chem. Soc. Jap., 46 (6), 1830, 1973
- G. CARDILLO, Gazz. Chim. Ital. 1966, 96, (8-9), 973.

Le ou les composés de formule (I) représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les paraaminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (II) suivante, et leurs sels d'addition avec un acide : 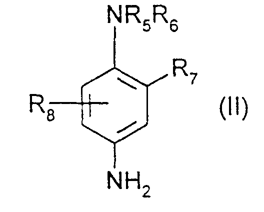 dans laquelle :
R₅ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄),
R₆ représente un atome d'hydrogène, un radical alkyle en C₁-C₄, monohydroxyalkyle en C₁-C₄ ou polyhydroxyalkyle en C₂-C₄,
R₇ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₈ représente un atome d'hydrogène ou un radical alkyle en C₁-C₄.

Dans la formule (II) ci-dessus, et lorsque R₇ est différent d'un atome d'hydrogène, alors R₅ et R₆ représentent de préférence un atome d'hydrogène et R₇ est de préférence identique à R₈, et lorsque R₇ représente un atome d'halogène, alors R₅, R₆ et R₈ représentent de préférence un atome d'hydrogène.

Parmi les paraphénylènediamines de formule (II) ci-dessus, on peut plus particulièrement citer la paraphénylènediamine, la paratoluylènediamine, la 2-isopropyl paraphénylène-diamine, la 2-β-hydroxyéthyl paraphénylènediamine, la 2-β-hydroxyéthyloxy para-phénylènediamine, la 2,6-diméthyl paraphénylènediamine, la 2,6-diéthyl paraphénylène-diamine, la 2,3-diméthyl paraphénylènediamine, la N,N-bis-(β-hydroxyéthyl) para-phénylènediamine, le 4-amino 1-(β-méthoxyéthyl)amino benzène, la 2-chloro para-phénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide : 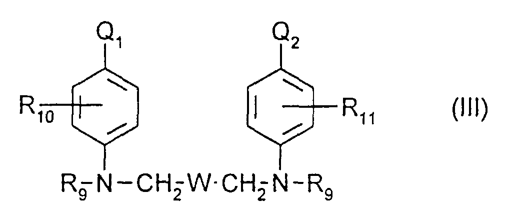 dans laquelle :
Q₁ et Q₂, identiques ou différents, représentent un radical hydroxyle ou NHR₁₂ dans lequel R₁₂ représente un atome d'hydrogène ou un radical alkyle en C₁-C₄,
R₉ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₀ et R₁₁, identiques ou différents, représentent un atome d'hydrogène ou d'halogène ou un radical alkyle en C₁-C₄,
W représente un radical pris dans le groupe constitué par les radicaux suivants :

   -(CH₂)ₙ ; -(CH₂)ₘ-O-(CH₂)ₘ; -(CH₂)ₘ-CHOH-(CH₂)ₘ

   et 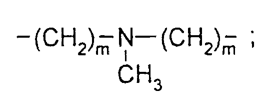
dans lesquels n est un nombre entier compris entre 0 et 8 inclusivement et m est un nombre entier compris entre 0 et 4 inclusivement.

Parmi les bis-phénylalkylènediamines de formules (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Parmi les paraaminophénols utilisables à titre de bases d'oxydation dans la composition tinctoriale selon l'invention, on peut notamment citer les composés répondant à la formule (IV) suivante, et leurs sels d'addition avec un acide : 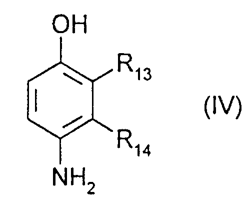 dans laquelle :
R₁₃ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₄ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les paraaminophénols de formule (IV) ci-dessus, on peut plus particulièrement citer le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triamino-pyrimidine, et leurs sels d'addition avec un acide.

Parmi les dérivés pyràzoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole et le 1-(4'-chlorobenzyl)-4,5-diaminopyrazole, et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des composés de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxybenzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau.

A titre de solvant organique, on peut par exemple citer les alcools inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. II peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 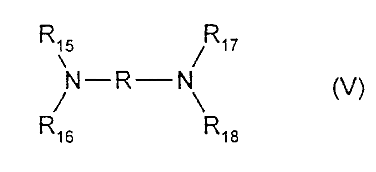 dans laquelle R est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des pyrazolin-3,5-diones de formule (I) ci-dessus, à titre de coupleur, en association avec au moins une base d'oxydation pour la teinture d'oxydation des fibres kératiniques et en particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus.

Ces dispositifs peuvent être équipés d'un moyen permettant de délivrer sur les cheveux le mélange souhaité, tel que les dispositifs décrits dans le brevet FR-2 586 913 au nom de la demanderesse.

### EXEMPLES

### EXEMPLES 1 ET 2 DE TEINTURE EN MILIEU ALCALIN

On a préparé les compositions tinctoriales, conformes à l'invention, suivantes (teneurs en grammes) :

| **EXEMPLE** | **1** | **2** |
|---|---|---|
| 1,2-diphényl pyrazolin-3,5-dione (coupleur) | 0,756 | - |
| 1,2-diéthyl pyrazolin-3,5-dione (coupleur) | - | 0,468 |
| Paraphénylènediamine | 0,324 | - |
| Dichlorhydrate de 1,3-diméthyl-4,5-diamino pyrazole (base d'oxydation) | - | 0,597 |
| Support de teinture commun | (*) | (*) |
| Eau déminéralisée q.s.p. | 100 g | 100 g |

| | | |
|---|---|---|
| **NB** : la 1,2-diphényl pyrazolin-3,5-dione et la 1,2-diéthyl pyrazolin-3,5-dione ont été préparées selon le procédé de synthèse décrit dans le brevet US 4 128 425. (*) Support de teinture commun : - Alcool benzylique 2,0 g - Polyéthylène glycol à 6 moles d'oxyde d'éthylène 3,0 g - Ethanol 20,0 g - Alkyl (C₈-C₁₀) polyglucoside en solution aqueuse à 60% de matière active, tamponné par du citrate d'ammonium, vendu sous la dénomination ORAMIX CG110 par la société SEPPIC 6,0 g - Ammoniaque à 20% de NH₃ 10,0 g - Métabisulfite de sodium 0,228 g - Agent séquestrant q.s. | | |

Au moment de l'emploi, chaque composition tinctoriale a été mélangée avec une quantité égale en poids d'une solution aqueuse de persulfate d'ammonium à 6.10⁻³ mole %.

Le mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans les nuances figurant dans le tableau ci-dessous:

| **Exemple** | **pH du mélange** | **Nuance obtenue sur cheveux gris naturels à 90% de blancs** | **Nuance obtenue sur cheveux gris à 90% de blancs permanentés** |
|---|---|---|---|
| **1** | 9,3 | Irisé rabattu | Irisé rabattu |
| **2** | 9,9 | Safran léger | Jaune safran |

## Revendications

1. Composition pour la teinture des fibres kératiniques et en particulier des fibres kératiniques humaines telles que les cheveux, **caractérisée par le fait qu'**elle comprend, dans un milieu approprié pour la teinture :
- à titre de coupleur, au moins un composé pyrazolin-3,5-dione de formule (I) suivante ou l'un de ses sels d'addition avec un acide : 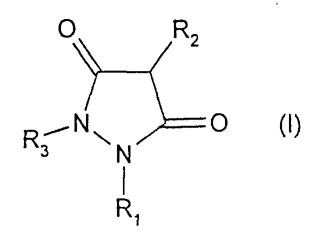
dans laquelle :
. R₂ représente : un atome d'hydrogène ; un atome d'halogène tel que brome, chlore ou fluor ; un groupe acétylamido ; un radical alcoxy ; un radical acyloxy ; un radical aryloxy ; un radical arylthio ; un radical alkylthio ; un radical hétéroarylthio ; un radical hétéroaryloxy ; un radical thiocyano ; un radical N,N-diéthyl thiocarbonylthio ; un radical dodécyloxythiocarbonylthio ; un radical benzènesulfonamido ; un radical N-éthyltoluène sulfonamido ; un radical pentafluorobutanamido ; un radical 2,3,4,5,6-penta-fluorobenzamido ; un radical p-cyanophényluréido, un radical N,N-diéthylsulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; un radical NR^{III}R^{IV} avec R^{III} et R^{IV} représentant, identiques ou différents, un alkyle en C₁-C₄ , un hydroxyalkyle ; un carboxyle ; ou un radical alcoxycarboxylique.
. R₁ et R₃ représentent un atome d'hydrogène ; un radical alkyle en C₁-C₅ linéaire ou ramifié ; un radical mono- ou poly-hydroxyalkyle en C₂-C₄ ; un radical aminoalkyle en C₂-C₄ ; un radical phényle ; un radical phényle substitué par un atome-d'halogène ou un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, méthylènedioxy ou amino ; ou un radical 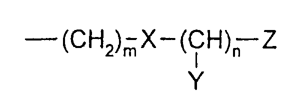 dans lequel m et n sont des nombres entiers, identiques ou différents, compris entre 1 et 3 inclusivement, X représente un atome d'oxygène ou bien le groupement NH, Y représente un atome d'hydrogène ou bien un radical méthyle, et Z représente un radical méthyle, un groupement OR ou NRR' dans lesquels R et R', qui peuvent être identiques ou différents, désignent un atome d'hydrogène, un radical méthyle ou un radical éthyle ; un radical amino ; un alkylamino en C₁-C₄ ; un radical carboxyle ; un radical alcoxycarbonyle ; un radical aryloxycarbonyle ; un hétérocycle à 5 ou 6 chaînons possédant au moins un atome d'azote, de soufre ou d'oxygène ; un radical acyle ; un groupe sulfonyle; un groupe phosphonyle;
étant entendu que lorsque R₂ et R₃ représentent simultanément un atome d'hydrogène, alors R₁ est différent d'un atome d'hydrogène, d'un radical alkyle en C₁-C₅ ou d'un radical phényle ;
- au moins une base d'oxydation.

2. Composition selon la revendication 1, **caractérisée par le fait que** les radicaux R₃ et R₁ de la formule (I) sont choisis dans le groupe constitué par :
hydrogène ; alkyle en C₁-C₄ ; mono- ou poly-hydroxyalkyle en C₂-C₄ ; aminoalkyle en C₂-C₄ ; phényle ; dialkylaminoalkyle ; phényle substitué par un chlore, un méthoxy, un nitro trifluorométhyle, amino, méthylamino ou un méthyle ; benzyle ; benzyle substitué par un chlore, méthoxy, méthyle ; alcoxycarbonyle ; aryloxycarbonyle ; pyridyle ; furyle ; thiènyle ; pyrrolyle ; thiazolyle ; acyle.

3. Composition selon la revendication 2, **caractérisée par le fait que** les radicaux R₁ et R₃ sont choisis dans le groupe constitué par :
hydrogène ; méthyle ; éthyle ; isopropyle ; 2-hydroxyéthyle ; 2-aminoéthyle ; phényle ; 2-, 3- ou 4-chlorophényle ; 3- ou 4-méthoxyphényle ; 3- ou 4-toluyle ; benzyle ; méthoxycarbonyle ; éthoxycarbonyle ; pyridyle ; pyrazolyle ; pyrrolyle.

4. Composition selon la revendication 2 ou 3, **caractérisée par le fait que** les radicaux R₁ et R₃ sont choisis dans le groupe constitué par :
hydrogène ; méthyle ; éthyle ; isopropyle ; phényle ; 4-chlorophényle ; 4-toluyle ; benzyle ; pyridyle ; pyrazolyle.

5. Composition selon l'une quelconque des revendications 1 à 4, **caractérisée par le fait que** les radicaux R₂ de la formule (I) sont choisis dans le groupe constitué par :
un atome d'hydrogène ; un alcoxy en C₁-C₄ ; phénoxy ; phénoxy substitué par un atome d'halogène, un alkyle en C₁-C₄, un carboxyle, un groupe trifluorométhyie ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄ , un hydroxyalkyle en C₁-C₄ ; un carboxyle ; un radical alcoxycarboxylique en C₁-C₄.

6. Composition selon la revendication 5, **caractérisée par le fait que** les radicaux R₂ de la formule (I) sont choisis dans le groupe constitué par :
hydrogène ; chlore ou brome ; méthoxy ou éthoxy ; phénoxy ; 4-méthylphénoxy ; acyloxy ; benzyloxy ; méthylthio ou éthylthio ; phénylthio ; 4-méthylphénylthio ; 2-tertio-butylphénylthio; acétamido ; phénylacétamido ; diméthylamino ; diéthylamino ; éthyl-méthylamino ; (β-hydroxyéthyl)méthylamino.

7. Composition selon la revendication 6, **caractérisée par le fait que** les radicaux R₂ de la formule (I) sont choisis dans le groupe constitué par :
hydrogène ; chlore ; éthoxy ; phénoxy ; benzyloxy ; acyloxy ; acétamido ; diméthylamino.

8. Composition selon l'une quelconque des revendications 1 à 7, **caractérisée par le fait que** les composés de formule (I) sont choisis parmi ceux pour lesquels :
- R₁ désigne hydrogène, méthyle, éthyle ou phényle ;
- R₂ désigne chlore ou éthoxy;
- R₃ désigne méthyle, éthyle ou phényle.

9. Composition selon la revendication 8, **caractérisée par le fait que** les composés de formule (I) sont choisis parmi :
- la 1,2-diphényl pyrazolin-3,5-dione,
- la 1,2-diéthyl pyrazolin-3,5-dione,
- la 1,2-diméthyl pyrazolin-3,5-dione,
- la 4-chloro-1,2-diéthyl pyrazolin-3,5-dione,
et leurs sels d'addition avec un acide.

10. Composition selon l'une quelconque des revendications précédentes, **caractérisée par le fait que** les sels d'addition avec un acide des composés de formule (I) sont choisis parmi les chlorhydrates, les bromhydrates, les sulfates, les tartrates, les benzènesulfonates, les lactates, les tosylates et les acétates.

11. Composition selon l'une quelconque des revendications précédentes, **caractérisée par le fait que** le ou les composés de formule (I) représentent de 0,0005 à 12 % en poids du poids total de la composition tinctoriale.

12. Composition selon la revendication 11, **caractérisée par le fait que** le ou les composés de formule (I) représentent de 0,0005 à 6 % en poids du poids total de la composition tinctoriale.

13. Composition selon l'une quelconque des revendications précédentes, **caractérisée par le fait que** la ou les bases d'oxydation sont choisies parmi les paraphénylènediamines, les bis-phénylalkylènediamines, les paraaminophénols, les orthoaminophénols, les bases hétérocycliques, et leurs sels d'addition avec un acide.

14. Composition selon l'une quelconque des revendications précédentes, **caractérisée par le fait que** la ou les bases d'oxydation représentent de 0,0005 à 12% en poids environ du poids total de la composition tinctoriale.

15. Composition selon la revendication 14, **caractérisée par le fait que** la ou les bases d'oxydation représentent de 0,005 à 6 % en poids environ du poids total de la composition tinctoriale.

16. Composition selon l'une quelconque des revendications précédentes, **caractérisée par le fait qu'**elle renferme en outre un ou plusieurs coupleurs additionnels différents des composés de formule (I) et/ou un ou plusieurs colorants directs.

17. Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcools inférieurs en C₁-C₄, le glycérol, les glycols et éthers de glycols, les alcools aromatiques, les produits analogues et leurs mélanges.

18. Composition selon l'une quelconque des revendications précédentes, **caractérisée par le fait qu'**elle présente un pH compris entre 3 et 12.

19. Composition selon l'une quelconque des revendications précédentes, **caractérisée par le fait qu'**elle se présente sous forme de liquides, de crèmes, de gels, ou sous toute autre forme appropriée pour réaliser une teinture des fibres kératiniques, et notamment des cheveux humains.

20. Utilisation des composés de formule (I) ou de leurs sels d'addition avec un acide tels que définis dans l'une quelconque des revendications 1 à 10, à titre de coupleurs dans des compositions pour la teinture d'oxydation des fibres kératiniques et en particulier des fibres kératiniques humaines telles que les cheveux, en association avec au moins une base d'oxydation.

21. Procédé de teinture d'oxydation des fibres kératiniques et en particulier des fibres kératiniques humaines telles que les cheveux, **caractérisé par le fait qu'**on applique sur ces fibres au moins une composition tinctoriale telle que définie à l'une quelconque des revendication 1 à 19, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

22. Procédé selon la revendication 21, **caractérisé par le fait que** l'agent oxydant est choisi parmi le peroxyde d'hydrogène, le peroxyde d'urée, les bromates de métaux alcalins, les persels tels que les perborates et persulfates.

23. Dispositif à plusieurs compartiments, ou "kit" de teinture à plusieurs compartiments, dont un premier compartiment renferme une composition tinctoriale telle que définie à l'une quelconque des revendications 1 à 19 et un second compartiment renferme une composition oxydante.

## Claims

1. Composition for dyeing keratin fibres, and in particular human keratin fibres such as the hair, **characterized in that** it comprises, in a medium that is suitable for dyeing:
- as coupler, at least one pyrazoline-3,5-dione of formula (I) below, or an addition salt thereof with an acid: 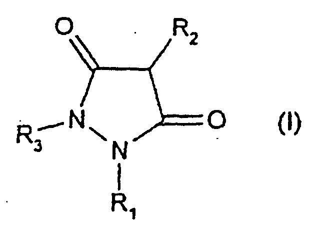 in which:
R₂ represents: a hydrogen atom; a halogen atom such as bromine, chlorine or fluorine; an acetylamido group; an alkoxy radical; an acyloxy radical; an aryloxy radical; an arylthio radical; an alkylthio radical; a heteroarylthio radical; a heteroaryloxy radical; a thiocyano radical; an N,N-diethylthiocarbonylthio radical; a dodecyloxythiocarbonylthio radical; a benzenesulphonamido radical; an N-ethyltoluenesulphonamido radical; a pentafluorobutanamido radical; a 2,3,4,5,6-pentafluorobenzamido radical; a p-cyanophenylureido radical; an N,N-diethylsulphamoylamino radical; a pyrazolyl radical; an imidazolyl radical; a triazolyl radical; a tetrazolyl radical; a benzimidazolyl radical; a 1-benzyl-5-ethoxy-3-hydantoinyl radical; a 1-benzyl-3-hydantoinyl radical; a 5,5-dimethyl-2,4-dioxo-3-oxazolidinyl radical; a 2-oxy-1,2-dihydro-1-pyridinyl radical; an alkylamido; an arylamido; a radical NR^{III}R^{IV} with R^{III} and R^{IV}, which may be identical or different, representing a C₁-C₄ alkyl, a hydroxyalkyl; a carboxyl; or an alkoxycarboxylic radical;
R₁ and R₃ represent a hydrogen atom; a linear or branched C₁-C₅ alkyl radical; a C₂-C₄ monohydroxyalkyl or polyhydroxyalkyl radical; a C₂-C₄ aminoalkyl radical; a phenyl radical; a phenyl radical substituted with a halogen atom or a C₁-C₄ alkyl, C₁-C₄ alkoxy, nitro, trifluoromethyl, amino or C₁-C₄ alkylamino radical; a benzyl radical; a benzyl radical substituted with a halogen atom or with a C₁-C₄ alkyl, C₁-C₄ alkoxy, methylenedioxy or amino radical; or a radical 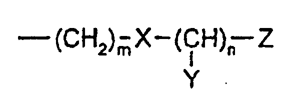 in which m and n are integers, which may be identical or different, between 1 and 3 inclusive, X represents an oxygen atom or an NH group, Y represents a hydrogen atom or a methyl radical, and Z represents a methyl radical, a group OR or NRR' in which R and R', which may be identical or different, denote a hydrogen atom, a methyl radical or an ethyl radical; an amino radical; a C₁-C₄ alkylamino; a carboxyl radical; an alkoxycarbonyl radical; an aryloxycarbonyl radical; a 5- or 6-membered heterocycle containing at least one nitrogen, sulphur or oxygen atom; an acyl radical; a sulphonyl group; a phosphonyl group;
it being understood that when R₂ and R₃ simultaneously represent a hydrogen atom, then R₁ is other than a hydrogen atom, a C₁-C₅ alkyl radical or a phenyl radical;
- at least one oxidation base.

2. Composition according to Claim 1, **characterized in that** the radicals R₃ and R₁ of formula (I) are chosen from the group consisting of:
hydrogen; C₁-C₄ alkyl; C₂-C₄ monohydroxyalkyl or polyhydroxyalkyl; C₂-C₄ aminoalkyl; phenyl; dialkylaminoalkyl; phenyl substituted with a chlorine, a methoxy, a nitro, trifluoromethyl, amino, methylamino or a methyl; benzyl; benzyl substituted with a chlorine, methoxy or methyl; alkoxycarbonyl; aryloxycarbonyl; pyridyl; furyl; thienyl; pyrrolyl; thiazolyl; acyl.

3. Composition according to Claim 2, **characterized in that** the radicals R₁ and R₃ are chosen from the group consisting of:
hydrogen; methyl; ethyl; isopropyl; 2-hydroxyethyl; 2-aminoethyl; phenyl; 2-, 3- or 4-chlorophenyl; 3- or 4-methoxyphenyl; 3- or 4-tolyl; benzyl; methoxycarbonyl; ethoxycarbonyl; pyridyl; pyrazolyl; pyrrolyl.

4. Composition according to Claim 2 or 3, **characterized in that** the radicals R₁ and R₃ are chosen from the group consisting of:
hydrogen; methyl; ethyl; isopropyl; phenyl; 4-chlorophenyl; 4-tolyl; benzyl; pyridyl; pyrazolyl.

5. Composition according to any one of Claims 1 to 4, **characterized in that** the radicals R₂ of formula (I) are chosen from the group consisting of:
a hydrogen atom; a C₁-C₄ alkoxy; phenoxy; phenoxy substituted with a halogen atom, a C₁-C₄ alkyl, a carboxyl or a trifluoromethyl group; an acyloxy radical; benzyloxy; C₁-C₄ alkylthio; phenylthio; phenylthio substituted with a halogen atom, a C₁-C₄ alkyl, a carboxyl or a trifluoromethyl group; a C₁-C₄ alkylamido; phenylamido; a radical NR^{III}R^{IV} with R^{III} and R^{IV}, which may be identical or different, representing a C₁-C₄ alkyl or a C₁-C₄ hydroxyalkyl; a carboxyl; a C₁-C₄ alkoxycarboxylic radical.

6. Composition according to Claim 5, **characterized in that** the radicals R₂ of formula (I) are chosen from the group consisting of:
hydrogen; chlorine or bromine; methoxy or ethoxy; phenoxy; 4-methylphenoxy; acyloxy; benzyloxy; methylthio or ethylthio; phenylthio; 4-methylphenylthio; 2-tert-butylphenylthio; acetamido; phenylacetamido; dimethylamino; diethylamino; ethylmethylamino; (β-hydroxyethyl)methylamino.

7. Composition according to Claim 6, **characterized in that** the radicals R₂ of formula (I) are chosen from the group consisting of:
hydrogen; chlorine; ethoxy; phenoxy; benzyloxy; acyloxy; acetamido; dimethylamino.

8. Composition according to any one of Claims 1 to 7, **characterized in that** the compounds of formula (I) are chosen from those for which:
- R₁ denotes hydrogen, methyl, ethyl or phenyl;
- R₂ denotes chlorine or ethoxy;
- R₃ denotes methyl, ethyl or phenyl.

9. Composition according to Claim 8, **characterized in that** the compounds of formula (I) are chosen from:
- 1,2-diphenylpyrazoline-3,5-dione,
- 1,2-diethylpyrazoline-3,5-dione,
- 1,2-dimethylpyrazoline-3,5-dione,
- 4-chloro-1,2-diethylpyrazoline-3,5-dione,
and the addition salts thereof with an acid.

10. Composition according to any one of the preceding claims, **characterized in that** the addition salts with an acid of the compounds of formula (I) are chosen from the hydrochlorides, hydrobromides, sulphates, tartrates, benzenesulphonates, lactates, tosylates and acetates.

11. Composition according to any one of the preceding claims, **characterized in that** the compound(s) of formula (I) represent(s) from 0.0005% to 12% by weight relative to the total weight of the dye composition.

12. Composition according to Claim 11, **characterized in that** the compound(s) of formula (I) represent(s) from 0.0005% to 6 % by weight relative to the total weight of the dye composition.

13. Composition according to any one of the preceding claims, **characterized in that** the oxidation base(s) is (are) chosen from para-phenylenediamines, bis(phenyl)alkylenediamines, para-aminophenols, ortho-aminophenols and heterocyclic bases, and the addition salts thereof with an acid.

14. Composition according to any one of the preceding claims, **characterized in that** the oxidation base(s) represent(s) from 0.0005% to 12% by weight approximately relative to the total weight of the dye composition.

15. Composition according to Claim 14, **characterized in that** the oxidation base(s) represent(s) from 0.005% to 6% by weight approximately relative to the total weight of the dye composition.

16. Composition according to any one of the preceding claims, **characterized in that** it also contains one or more additional couplers other than the compounds of formula (I) and/or one or more direct dyes.

17. Composition according to any one of the preceding claims, **characterized in that** the medium that is suitable for dyeing (or support) consists of water or a mixture of water and at least one organic solvent chosen from C₁-C₄ lower alcohols, glycerol, glycols and glycol ethers, aromatic alcohols, similar products and mixtures thereof.

18. Composition according to any one of the preceding claims, **characterized in that** it has a pH of between 3 and 12.

19. Composition according to any one of the preceding claims, **characterized in that** it is in the form of liquids, creams or gels, or in any other form that is suitable for dyeing keratin fibres, and especially human hair.

20. Use of the compounds of formula (I) or of the addition salts thereof with an acid as defined in any one of Claims 1 to 10, as couplers in compositions for the oxidation dyeing of keratin fibres, and in particular of human keratin fibres such as the hair, in combination with at least one oxidation base.

21. Process for the oxidation dyeing of keratin fibres, and in particular of human keratin fibres such as the hair, **characterized in that** at least one dye composition as defined in any one of Claims 1 to 19 is applied to these fibres, the colour being developed at acidic, neutral or alkaline pH with the aid of an oxidizing agent that is added just at the time of use to the dye composition, or that is present in an oxidizing composition applied simultaneously or sequentially in a separate manner.

22. Process according to Claim 21, **characterized in that** the oxidizing agent is chosen from hydrogen peroxide, urea peroxide, alkali metal bromates, and persalts such as perborates and persulphates.

23. Multi-compartment device, or multi-compartment dyeing "kit", a first compartment of which contains a dye composition as defined in any one of Claims 1 to 19, and a second compartment of which contains an oxidizing composition.

## Patentansprüche

1. Zusammensetzung zum Färben von Keratinfasern und insbesondere menschlichen Keratinfasern, wie dem Haar, **dadurch gekennzeichnet, daß** sie in einem zum Färben geeigneten Medium enthält:
- als Kuppler mindestens ein Pyrazolin-3,5-dion der folgenden Formel (I) oder mindestens ein Additionssalz dieser Verbindungen mit einer Säure: 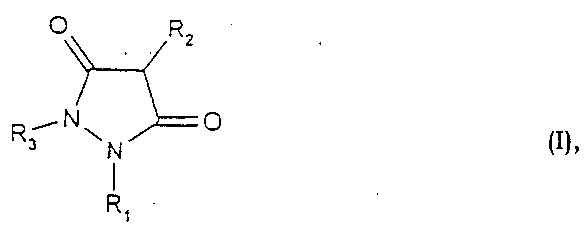
worin bedeuten :
- R₂ Wasserstoff; ein Halogenatom, wie Brom, Chlor oder Fluor; Acetylamido; Alkoxy; Acyloxy; Aryloxy; Arylthio; Alkylthio; Heteroarylthio; Heteroaryloxy; Thiocyano; N,N-Diethylthiocarbonylthio; Dodecyloxythiocarbonylthio; Benzolsulfonamido; N-Ethyltoluolsulfonamido; Pentafluorbutanamido; 2,3,4,5,6-Pentafluorbenzamido; p-Cyanophenylureido; N,N-Diethylsulfamoylamino; Pyrazolyl; Imidazolyl; Triazolyl; Tetrazolyl; Benzimidazolyl; 1-Benzyl-5-ethoxy-3-hydantoinyl; 1-Benzyl-3-hydantoinyl; 5,5-Dimethyl-2,4-dioxo-3-oxazolidinyl; 2-Oxy-1,2-dihydro-1-pyridinyl; Alkylamido; Arylamido; NR^{III}R^{IV}, worin R^{III} und R^{IV}, die identisch oder voneinander verschieden sind, C₁₋₄-Alkyl oder C₁₋₄-Hydroxyalkyl bedeuten; Carboxy; AIkoxycarboxy;
- die Gruppen R₁ und R₃ Wasserstoff; eine geradkettige oder verzweigte C₁₋₅-Alkylgruppe; C₂₋₄-Mono- oder Polyhydroxyalkyl; C₂₋₄-Aminoalkyl; Phenyl; eine Phenylgruppe, die mit einem Halogenatom, C₁₋₄-Alkyl, C₁₋₄-Alkoxy, Nitro, Trifluormethyl, Amino oder C₁₋₄-Alkylamino substituiert ist; Benzyl; eine Benzylgruppe, die mit einem Halogenatom, C₁₋₄-Alkyl, C₁₋₄-Alkoxy, Methylendioxy oder Amino substituiert ist; oder eine Gruppe 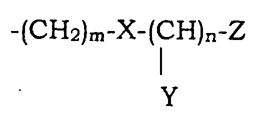 worin m und n, die identisch oder voneinander verschieden sind, ganze Zahlen im Bereich von 1 bis 3 bedeuten; X Sauerstoff oder die Gruppe NH bedeutet; Y Wasserstoff oder Methyl ist; und Z Methyl, OR oder NRR', wobei R und R', die identisch oder voneinander verschieden sein können, Wasserstoff, Methyl oder Ethyl bedeuten; Amino; C₁₋₄-Alkylamino; Carboxy; Alkoxycarbonyl; Aryloxycarbonyl; einen 5- oder 6-gliedrigen Heterocyclus, der mindestens ein Stickstoff-, Schwefel- oder Sauerstoffatom; Acyl; Sulfonyl; oder Phosphonyl bedeutet;
mit der Maßgabe, daß R₁ von Wasserstoff, C₁₋₅-Alkyl oder Phenyl verschieden ist, wenn R₂ und R₃ gleichzeitig Wasserstoff bedeuten;
und
mindestens eine Oxidationsbase.

2. Zusammensetzung nach Anspruch 1, **dadurch gekennzeichnet, daß** die Gruppen R₁ und R₃ der Formel (I) ausgewählt sind unter: Wasserstoff; C₁₋₄-Alkyl; C₂₋₄-Mono- oder Polyhydroxyalkyl; C₂₋₄-Aminoalkyl; Phenyl; Dialkylaminoalkyl; Phenylgruppen, die mit Chlor, Methoxy, Nitro, Trifluormethyl, Amino, Methylamino oder Methyl substituiert sind; Benzyl; Benzylgruppen, die mit Chlor, Methoxy oder Methyl substituiert sind; Alkoxycarbonyl; Aryloxycarbonyl; Pyridyl; Furyl; Thienyl; Pyrrolyl; Triazolyl; Acyl.

3. Zusammensetzung nach Anspruch 2, **dadurch gekennzeichnet, daß** die Gruppen R₁ und R₃ ausgewählt sind unter: Wasserstoff; Methyl; Ethyl; Isopropyl; 2-Hydroxyethyl; 2-Aminoethyl; Phenyl; 2-, 3- oder 4-Chlorphenyl; 3- oder 4-Methoxyphenyl; 3- oder 4-Tolyl; Benzyl; Methoxycarbonyl; Ethoxycarbonyl; Pyridyl; Pyrazolyl; und Pyrrolyl.

4. Zusammensetzung nach Anspruch 2 oder 3, **dadurch gekennzeichnet, daß** die Gruppen R₁ und R₃ ausgewählt sind unter: Wasserstoff; Methyl; Ethyl; Isopropyl; Phenyl; 4-Chlorphenyl; 4-Tolyl; Benzyl; Pyridyl; Pyrazolyl.

5. Zusammensetzung nach einem der Ansprüche 1 bis 4, **dadurch gekennzeichnet, daß** die Gruppen R₂ der Formel (I) ausgewählt sind unter: Wasserstoff; C₁₋₄- Alkoxy; Phenoxy; Phenoxygruppen, die mit einem Halogenatom, C₁₋₄-Alkyl, Carboxy oder Trifluormethyl substituiert sind; Acyloxy; Benzyloxy; C₁₋₄-Alkylthio; Phenylthio; Phenylthiogruppen, die mit einem Halogenatom, C₁₋₄-Alkyl, Carboxy oder Trifluormethyl substituiert sind; C₁₋₄-Alkylamido; Phenylamido; NR^{III}R^{IV}, worin R^{III} und R^{IV}, die identisch oder voneinander verschieden sind, C₁₋₄-Alkyl oder C₁₋₄-Hydroxyalkyl bedeuten; Carboxy oder C₁₋₄-Alkoxycarboxy.

6. Zusammensetzung nach Anspruch 5, **dadurch gekennzeichnet, daß** die Gruppen R₂ der Formel (I) ausgewählt sind unter: Wasserstoff; Chlor oder Brom; Methoxy oder Ethoxy; Phenoxy; 4-Methylphenoxy; Acyloxy; Benzyloxy; Methylthio oder Ethylthio; Phenylthio; 4-Methylphenylthio; 2-t-Butylphenylthio; Acetamido; Phenylacetamido; Dimethylamino; Diethylamino; Ethylmethylamino; (β-Hydroxyethyl)-methylamino.

7. Zusammensetzung nach Anspruch 6, **dadurch gekennzeichnet, daß** die Gruppen R₂ der Formel (I) ausgewählt sind unter: Wasserstoff; Chlor; Ethoxy; Phenoxy; Benzyloxy; Acyloxy; Acetamido; und Dimethylamino.

8. Zusammensetzung nach einem der Ansprüche 1 bis 7, **dadurch gekennzeichnet, daß** die Verbindungen der Formel (I) unter den Verbindungen ausgewählt sind, worin bedeuten:
R₁ Wasserstoff, Methyl, Ethyl oder Phenyl;
R₂ Chlor oder Ethoxy;
R₃ Methyl, Ethyl oder Phenyl.

9. Zusammensetzung nach Anspruch 8, **dadurch gekennzeichnet, daß** die Verbindungen der Formel (I) ausgewählt sind unter:
- 1,2-Diphenyl-pyrazolin-3,5-dion;
- 1,2-Diethyl-pyrazolin-3,5-dion;
- 1,2-Dimethyl-pyrazolin-3,5-dion;
- 4-Chlor-1,2-diethyl-pyrazolin-3,5-dion; und
- deren Additionssalze mit einer Säure.

10. Zusammensetzung nach einem der vorhergehenden Ansprüche, **dadurch gekennzeichnet, daß** die Additionssalze der Verbindungen der Formel (I) mit einer Säure unter den Hydrochloriden, Hydrobromiden, Sulfaten, Tartraten, Benzolsulfonaten, Lactaten, Tosylaten und Acetaten ausgewählt sind.

11. Zusammensetzung nach einem der vorhergehenden Ansprüche, **dadurch gekennzeichnet, daß** die Verbindung(en) der Formel (I) 0,0005 bis 12 Gew.-% des Gesamtgewichts der Farbmittelzusammensetzung ausmachen.

12. Zusammensetzung nach Anspruch 11, **dadurch gekennzeichnet, daß** die Verbindung(en) der Formel (I) 0,005 bis 6 Gew.-% des Gesamtgewichts der Farbmittelzusammensetzung ausmachen.

13. Zusammensetzung nach einem der vorhergehenden Ansprüche, **dadurch gekennzeichnet, daß** die Oxidationsbase(n) unter den p-Phenylendiaminen, Bisphenylalkylendiaminen, p-Aminophenolen, o-Aminophenolen, heterocyclischen Basen und deren Additionssalzen mit einer Säure ausgewählt sind.

14. Zusammensetzung nach einem der vorhergehenden Ansprüche, **dadurch gekennzeichnet, daß** die Oxidationsbase(n) etwa 0,0005 bis 12 Gew.-% des Gesamtgewichts der Farbmittelzusammensetzung ausmachen.

15. Zusammensetzung nach Anspruch 14, **dadurch gekennzeichnet, daß** die Oxidationsbase(n) etwa 0,005 bis 6 Gew.-% des Gesamtgewichts der Farbmittelzusammensetzung ausmachen.

16. Zusammensetzung nach einem der vorhergehenden Ansprüche, **dadurch gekennzeichnet, daß** sie ferner einen oder mehrere zusätzliche Kuppler, die von den Verbindungen der Formel (I) verschieden sind, und/oder einen oder mehrere Direktfarbstoffe enthält.

17. Zusammensetzung nach einem der vorhergehenden Ansprüche, **dadurch gekennzeichnet, daß** das zum Färben geeignete Medium (oder der Träger) aus Wasser oder einem Gemisch von Wasser und mindestens einem organischen Lösungsmittel besteht, das unter den niederen C₁₋₄-Alkoholen, Glycerin, Glykolen und Glykolethern, aromatischen Alkoholen, analogen Produkten und deren Gemischen ausgewählt ist.

18. Zusammensetzung nach einem der vorhergehenden Ansprüche, **dadurch gekennzeichnet, daß** sie einen pH-Wert im Bereich von 3 bis 12 aufweist.

19. Zusammensetzung nach einem der vorhergehenden Ansprüche, **dadurch gekennzeichnet, daß** sie als Flüssigkeit, Creme oder Gel oder in beliebigen weiteren Formen, die zur Durchführung einer Färbung von Keratinfasern und insbesondere von menschlichem Haar geeignet sind, vorliegt.

20. Verwendung von Verbindungen der Formel (I) oder von Additionssalzen dieser Verbindungen mit einer Säure nach einem der Ansprüche 1 bis 10 als Kuppler in Zusammensetzungen zum oxidativen Färben von Keratinfasern und insbesondere menschlichen Keratinfasern, wie dem Haar, in Kombination mit mindestens einer Oxidationsbase.

21. Verfahren zum oxidativen Färben von Keratinfasern und insbesondere menschlichen Keratinfasern, wie dem Haar, **dadurch gekennzeichnet, daß** auf die Fasern mindestens eine Farbmittelzusammensetzung nach einem der Ansprüche 1 bis 19 aufgetragen wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

22. Verfahren nach Anspruch 21, **dadurch gekennzeichnet, daß** das Oxidationsmittel unter Wasserstoffperoxid, Harnstoffperoxid, Alkalimetallbromaten und Salzen von Persäuren, wie Perboraten und Persulfaten, ausgewählt ist.

23. Vorrichtung mit mehreren Abteilungen oder "Kit" zum Färben mit mehreren Abteilungen, wobei eine erste Abteilung eine Farbmittelzusammensetzung nach einem der Ansprüche 1 bis 19 und eine zweite Abteilung eine oxidierende Zusammensetzung enthält.
